(19) Europäisches Patentamt
European Patent Office
Office européen des brevets

(11) Publication number : **0 569 141 A2**

# (12) EUROPEAN PATENT APPLICATION

(21) Application number : **93302894.6**

(51) Int. Cl.$^5$ : **A61K 39/395**

(22) Date of filing : **14.04.93**

The applicant has subsequently filed a sequence listing and declared, that it includes no new matter.

(30) Priority : **14.04.92 US 868983**

(43) Date of publication of application : **10.11.93 Bulletin 93/45**

(84) Designated Contracting States : **AT BE CH DE DK ES FR GB GR IE IT LI LU NL PT SE**

(71) Applicant : **HYBRITECH INCORPORATED**
**11095 Torreyana Road**
**San Diego California 92126 (US)**

(72) Inventor : **Grauer, Lana Suzanne**
**13252 Caminito Point Del Mar**
**Del Mar, California 92014 (JP)**

(74) Representative : **Hudson, Christopher Mark et al**
**Erl Wood Manor**
**Windlesham Surrey GU20 6PH (GB)**

(54) **Methods of inhibiting the growth of multidrug resistant tumors with an antibody.**

(57) This invention provides non-complement or non-ADCC mediated methods for inhibiting in vivo proliferation of tumor cells expressing a multidrug resistant p-glycoprotein. Such methods are useful for treating patients having a disorder associated with tumor cells expressing such multidrug resistant p-glycoproteins.

EP 0 569 141 A2

The present invention generally relates to multi-drug resistance exhibited by many tumor cells. More specifically, the present invention relates to the use of multi-drug resistant (MDR) antibodies to inhibit the growth of such tumor cells.

This application is a continuation-in-part of U.S. Patent Application Serial No. 07/732,969, filed on July 19, 1991, which is a continuation-in-part of U.S. Patent Application Serial No. 07/491,406 filed on March 9, 1990, both of which are incorporated herein by reference in their entirety.

The ability of certain tumor cells to acquire multi-drug resistance has been a significant barrier to the effective use of chemotherapy in the treatment of such tumors and other neoplastic disorders. Multi-drug resistance is the phenomenon by which certain cancer cells selected for resistance to one drug can simultaneously exhibit resistance to structurally and functionally unrelated drugs to which they have not been previously exposed.

Cells exhibiting the multi-drug resistance phenotype show reduced intracellular accumulation of such drugs compared with the corresponding drug-sensitive cells. A common characteristic of many MDR cells is the overexpression of cell surface P-glycoproteins that are absent or expressed in low levels in drug-sensitive cells. One function of the P-glycoproteins is to act as a drug efflux pump by which intracellular toxins are actively pumped out of the cell. It is believed certain regions of the P-glycoprotein bind to the energy-carrying compound, ATP, which provides the energy to pump the non-related toxins out of the cell.

At least two P-glycoprotein genes have been identified in humans and three in mice, suggesting that a family of such glycoproteins may exist. One human P-glycoprotein has an apparent molecular weight of about 180 Kd, while the other has an apparent molecular weight of about 170 Kd. Monoclonal antibodies specifically reactive with these P-glycoproteins are known and have been described, for example, in Hamada & Tsuruo, PNAS (U.S.A.) 83:7785-7789 (1986) and in European Patent Application No. 87309939.4, published on May 18, 1988. These monoclonal antibodies are commonly referred to as anti-MDR antibodies.

A variety of studies have been published which describe inhibition of human tumor xenografts or killing of tumor cells in culture in the presence of monoclonal antibodies directed against molecules on the surface of the tumor cells. Such studies are reported in Dillman, CRC Crit. Rev. Oncol. Hematol., 1:357-385 (1984); Dillman et al., J. Clin. Oncol. 2:881 (1984); Miller et al., Lancet 2:228 (1981); Dillman et al., Blood 59, 1036 (1984); Foon et al., Blood 84, 1085 (1984); Houghton et al., Proc. Natl. Acad. Sci. USA. 82:1242 (1985); and Sears et al., J. Biol. Resp. Modif. 3:138 (1983). All of these studies site ADCC (antibody directed cell cytotoxicity) or complement mediated cell lysis as the mechanism for tumor growth inhibition as reported, for example, in Tsuruo et al., Jpn. J. Can. Res. 80:627-631 (1989). In ADCC, the action of the antibody occurs through Fc receptors. Platelets, neutrophils, eosinophils and cells of the mononuclear phagocyte series all have receptors for Fc, by which they engage target tissues. K cells which mediate ADCC are functionally defined Fc receptor$^{+}$ cytotoxic cells. CLq is a soluble Fc receptor and the first molecule of the complement classical pathway. Activation of complement C3 can generate complement-mediated lytic damage to target cells directly, and also allows phagocytic cells with receptors for activated C3 to bind to the target.

A need exists for mechanisms other than complement or ADCC mediated inhibition of tumor growth. The present invention satisfies this need and provides related advantages as well.

The present invention provides methods for treating a patient having a disorder associated with tumor cells expressing a multidrug resistant p-glycoprotein by administering to the patient a therapeutically effective amount of a specific binding agent of the invention in which the inhibition is non-complement or non-ADCC mediated. The specific binding agent can be an antibody or fragments thereof that are specifically reactive with such p-glycoproteins.

Figure 1 shows the effect of anti-p-glycoprotein monoclonal antibodies LS2H241, LS2L162, and the Fab'3 fragment $(LS2H)_2$XCHA on the growth of $2780^{AD}10$ xenografts.

Figure 2 shows the effect of anti-p-glycoprotein monoclonal antibodies LS2H241, LS2L162 and BC4E549, and the fragment Fab'2 on growth of $2780^{AD}10$ xenografts. The results were obtained by injecting mice in groups of 10 with 100µg of monoclonal antibodies on day 2 and day 7 after innoculation of tumor cells.

Figure 3 shows the effect of LS2L162 on ovarian tumor A2780 in athymic nude mice. The results were obtained by injecting 500µg antibody or saline on days 2 and 7 post tumor implantation.

Figure 4 shows inhibition of human drug resistant colon carcinoma Colo320DM by LS2L162 in athymic nude mice. The results were obtained by injecting saline or 500µg antibody given on days 2 and 7 after tumor implantation. Nonpalpable mice are not included in calculations for growth curves.

Figure 5 shows carbohydrate staining of LS2H241 and fragments of LS2L162 and LS2H241 with Ponceau S. The results show that the antibody fragments are fully digested. The bands identified are the correct molecular weights for heavy and light chain components of Fab' and $Fab'_2$.

Figure 6 shows carbohydrate staining of LS2H241 and fragments of LS2L162 and LS2H241 with Con A/HRP.

The present invention provides methods of inhibiting the in vivo proliferation of tumor cells expressing multidrug resistant P-glycoproteins. The methods are accomplished by contacting tumor cells expressing the MDR P-glycoproteins with a specific binding agent having specific reactivity with the P-glycoprotein in which the inhibition is not mediated by complement or ADCC.

As used herein, the term "specific binding agent" refers to any molecule that has specific reactivity with the MDR P-glycoprotein described above. Preferably, it is an antibody, but may also be a chemical compound capable of binding with the p-glycoprotein. The agents can be polyclonal or monoclonal antibodies such as, for example, LS2H241, LS2L162 or other described in Rittman-Grauer et al., Can. Res. (April 1, 1992).

The term "specific binding agent" also includes any reactive fragment or fragments of antibodies such as Fab molecules, Fab proteins, single chain polypeptides or the multi-functional antibodies disclosed in U.S. Serial No. 07/732,969, such as $(LS2H241)_2XCHA$, having binding affinity for the antigen of the present invention. As used herein, the term "Fab" or "fragment" refers to regions of antibody molecules which include the variable portions of the heavy chain and/or light chain and which exhibit binding activity. "Fab protein" includes aggregates of one heavy and one light chain (commonly known as Fab), as well as tetrameres which correspond to the two branch segments of the antibody Y (commonly known as $F(ab)_2$), whether any of the above are covalently or non-covalently aggregated so long as the aggregation is capable of selectively reacting with a particular antigen or antigen family. Such fragments include, for example, $LS2H241(Fab)'_2$ or $LS2LI62(Fab)'_2$.

The specific binding agents of the invention may be produced by any method known in the art. For example, polyclonal and monoclonal antibodies as well as various fragments thereof can be produced by methods well known in the art as described, for instance, in Harlow and Lane, Antibodies: A Laboratory Manual, 626-631 (1988), and in Rittman-Grauer et al., Can. Res. (April 1, 1992), both of which are incorporated herein by reference. Such agents may also be produced synthetically by methods known in the art or recombinantly by methods described for example in Maniatis et al., Molecular Cloning: A Laboratory Manual (Cold Spring Harbor Laboratory 1989), incorporated herein by reference.

The specific bindinq agents of the present invention can also be humanized or CDR grafted antibodies prepared by methods known in the art. For example, CDR-grafted antibody heavy and light chains containing acceptor framework and donor antigen binding regions can be prepared as described in United Kingdom Patent Application GB 2246570A, filed on December 21, 1990, which is incorporated herein by reference.

As used in the methods of the present invention, the specific binding agents can be administered to a patient by any means known to those skilled in the art, including parenteral injection or topical application. Injection can be done intravascularly, intraperitoneally, subcutaneously or intramuscularly. For parenteral administration, the specific binding agents can be administered in admixture with a suitable pharmaceutically acceptable carrier.

The dosage regimen for treating MDR-associated disorders depends on a variety of factors, including the type of disorder, age, weight, sex and medical condition of the patient, the severity of the condition, the route of administration and the type of diagnostic or therapeutic agent used. A skilled physician can readily determine and prescribe the effective amount of the compound or pharmaceutical composition required to diagnose or treat the patient. Conventionally, one skilled in the art would employ relatively low doses initially and subsequently increase the dose until a maximum response is obtained. Because the trifunctional compound of the present invention specifically localizes the agent, much lower doses are believed to be effective than with conventional methods. For example, an effective dose of the specific binding agent can be up to about 2-5 mg per 20 grams of body weight, and preferably in the range of about 100μg to about 500μg per 20 grams of body weight.

The type of in vivo tumor inhibition which is identified with the anti-p-glycoprotein antibodies herein represents a novel form of antibody-mediated tumor inhibition which is not ADCC or complement related. Complete removal of the Fc portion of the antibody which contains the complement binding site does not abrogate the ability of the antibody to inhibit tumor growth in vivo as shown in the examples below. The precise mechanism responsible for the tumor cell growth inhibition has not been identified. Although not wishing to be limited to any particular theory, it is believed that the mechanism may be related to, (a) functional blocking of the p-glycoprotein pump by the anti-p-glycoprotein antibodies which could result in accumulation of a cytotoxic substance in the cells, (b) blockage of the ability of the multidrug resistant cells to adhere to the extracellular matrix or (c) the mechanism may be related to recent findings by Chin et al., Science 255:459 (1992), which indicate that the promoter of the human MDRI gene is also a target for the c-Ha-Ras-I oncogene and the p53 tumor suppressor gene products, both of which are associated with tumor progression.

The following examples are intended to illustrate but not limit the invention.

**EXAMPLE I**

GROWTH INHIBITION OF MULTIDRUG RESISTANT TUMOR, $2780^{AD}10$ IN NUDE MICE WITH ANTI-P-GLYCOPROTEIN ANTIBODIES

$2780^{AD}10$ cells (1 x $10^7$ cells/animal) were injected subcutaneously (s.c.) into the flanks of 6-8-week old female nude mice (obtained from Harlan Sprague Dawley, Indianapolis, IN). The $2780^{AD}10$ cell line was obtained from Dr. R.F. Ozols (Fox Chase Cancer Center). The $2780^{AD}10$ cell line is described in Rogan et al., Science 224:994 (1984). It is a multidrug resistant ovarian carcinoma line which has been shown to express p-glycoprotein. On days 2 and 7 following injections of the tumor cells, mice were injected i.v. with either 100μg or 500μg of intact monoclonal antibody or fragments. The intact monoclonal antibodies included LS2H241($IgG_1$), also referred to as HYB241, and LS2L162 ($IgG_{2b}$), also referred to as HYB162, two anti-p-glycoprotein antibodies, PSA399(IgG2b) an anti-prostate specific antigen antibody, and SI (IgGl), an antibody of unknown reactivity. The PSA399 and S1 antibodies served as isotype matched irrelevant antibody controls. The fragments included $(LS2H241)_2$XCHA and LS2H241F(ab)'$_2$, both are described in U.S. Serial No. 07/732,969, incorporated herein by reference. Groups of control mice received i.v. injections of saline. Tumor size was measured 2-3 times weekly.

**EXAMPLE II**

ANALYSIS OF FRAGMENTS

The fragments described in Example I were analyzed by SDS-PAGE electrophoresis under native and reduced conditions according to the method of Laemmli et al. Nature 227:680 (1970), incorporated herein by reference. Gels were stained for protein by immersing gels in 0.2% Ponceau S (Boehringer-Mannheim, Indianapolis, IN) stain in 3% trichloroacetic acid for 5 minutes and staining in distilled water. This method was used to ensure that digestion of the antibodies had been complete and that the Fc region had been deleted from the antibody molecule. As shown in Figure 6 the Fab' molecule produced a single protein band at 25kd and the Fab'$_2$ molecule produced a band at 25kd and 28kd.

As a further test to ensure that the Fc region and more specifically the complement binding regions of the antibody had been removed by the proteolytic digestion, the fragments were examined for their ability to be recognized by carbohydrate specific lectins. Samples of intact antibodies and fragments (5μg) which had been reduced with 5% β-mercaptoethanol were electrophoresed on 12% polyacrylamide gels in the presence of 0.1% SDS. Gels were blotted onto nitrocellulose, as described in Towbin et al. Proc. Natl. Aca. Sci. 76:4350 (1976), and the blots were blocked overnight in 1% bovine serum albumen in PBS. The blot was then incubated with Concanavillin A (2.5μg/ml) in phosphate buffered saline (PBS) for 1 hour. Following 3 washes in PBS the blot was incubated in horseradish peroxidase (5μg/ml) in 0.05M tris-HCl, 0.15M NaCl, lmM $MnCl_2$, 1mM $CaCl_2$, 1mM $MgCl_2$, pH 7.5 (Buffer 1) for 1 hour. Following two washes in tris buffered saline 0.05M tris-HCl, 0.15M NaCl pH 7.5 (TBS), the blot was stained with 0.6% 4-chloro-1-napthol in TBS with 0.018% hydrogen peroxide.

The heavy and light chains of HYB162 Fab' migrated as one band at 25kd (Figure 5). This band failed to stain with the Con A lectin and produced a negative image on the blot (Figure 6). Similarly, HYB241 Fab'$_2$ migrated as two bands on the gel at 28kd and 25kd (Figure 5) and also failed to stain with Con A (Figure 6). This negative staining indicates that there is no oligosaccharide present on the heavy or light chains at either HYB241 Fab'$_2$ or HYB162 Fab'. In contrast, HYB241 migrates as a two bands (Figure 5). The heavy chain (50kd) is glycosylated since it stains with the lectin, while the light chain at 25kd is nonglycosylated (Figure 6).

**EXAMPLE III**

SEQUENCE OF LS2H241 HEAVY AND LIGHT CHAIN VARIALLE REGIONS

The sequences of the heavy and light chain variable regions of LS2H241 were determined by sequence analysis on a Dupont Genesis 2000 DNA Sequencer. Sequences of LS2H241 heavy and light chain variable regions are provided in Tables 1 and 2. The Tables show the framework and CDR regions of the chains as well as various restriction sites and the amino acid sequences encoded by the indicated nucleotide sequences. The boxed region at the beginning of Framework 1 of the LS2H241 heavy variable region represents the oligonucleotide sequence used in cloning. The starred (*) bases represent the ambiguous/mixed species within the oligonucleotide.

Table 1

LS2H241 HEAVY CHAIN VARIABLE

FR1 | CDR1 | DPRPAU21 | PSS1

GAGGTSMAGCTKMAGSAGTCTGGGGGAGGCTTAGTGAAGCCTGGAGGGTCCCTGAAACTCTCCTGTGTAGCCTCTGGATTCAGTTTCAGTAGATATACCA 100
CTCCASKTCGAMKTCSTCAGACCCCCTCCGAATCACTTCGGACCTCCCAGGGACTTTGAGAGGACACATCGGAGACCTAAGTCAAAGTCATCTATATGGT
e v ? l ? ? s g g g l v k p g g s l k l s c v a s g f s f s r y t m

PFI1 | FR2 | BPDLPE21 | MBO2 | CDR2 | FR3

TGTCTTGGGTTCGCCAGACTCCGGAGAAGAGGCTGGAGTGGGTCGCAACCATTAGTAGTAGTGGTTATAATACCTACTATCTTGACAGTGTGAAGGGTCG 200
ACAGAACCCAAGCGGTCTGAGGCCTCTTCTCCGACCTCACCCAGCGTTGGTAATCATCATCACCAATATTATGGATGATAGAACTGTCACACTTCCCAGC
s w v r q t p e k r l e w v a t i s s s g y n t y y l d s v k g r

HPH1 | BSP1 | ECRV | CDR3

ATTCACCATCTCCAGAGACAATGCCAAGCAAAACCTGTACCTGCAAATGAACAGTCTGAGGTCTGAGGACACGGCCTTGTATTACTGTACAAGATATCAT 300
TAAGTGGTAGAGGTCTCTGTTACGGTTCGTTTTGGACATGGACGCTTACTTGTCAGACTCCAGACTCCTGTGCCGGAACATAATGACATGTTCTATAGTA
f t i s r d n a k q n l y l q m n s l r s e d t a l y y c t r y h

AHA2 | HGA1 | FR4 (JH9) | STY1 | BSTE | HGA1 | HPH1 | PST1 | Cγ | BBV1

AGCTACGACGCCTGGTTTGCTTACTGGGGCCAAGGGACGCTGGTCACCGTCTCTGCAGCCAAAACGACACCCCCATCTGT 380
TCGATGCTGCGGACCAAACGAATGACCCCGGTTCCCTGCGACCAGTGGCAGAGACGTCGGTTTTGCTGTGGGGGTAGACA
s y d a w f a y w g q g t l v t v s a a k t t p p s v

Table 2

LS2H241 LIGHT CHAIN VARIABLE

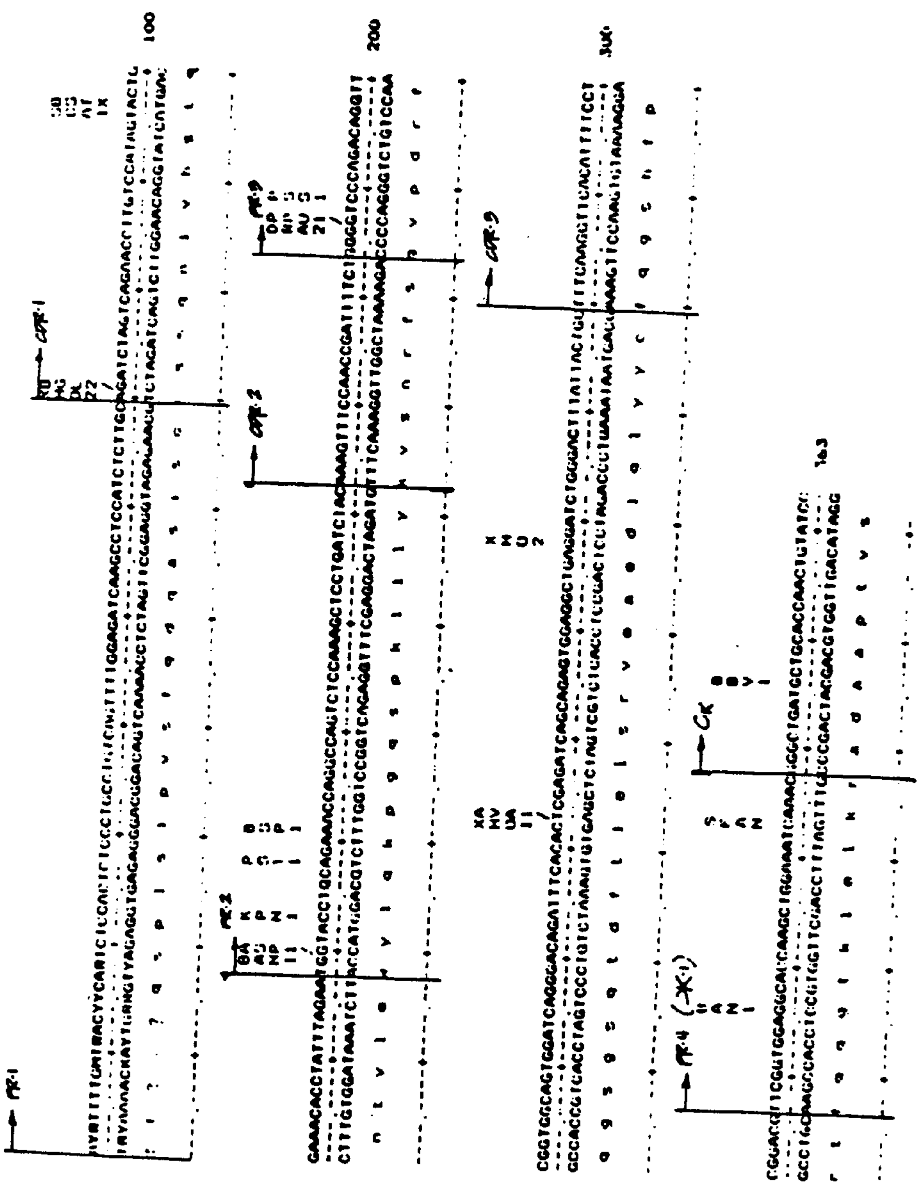

## EXAMPLE IV

INHIBITION OF MULTIDRUG-RESISTANT HUHAN OVARIAN CARCINOMA GROWTH IN ATHYMIC MICE BY $(LS2H241)_2XCHA$ TRIVALENT ANTIBODY

Balb c/nu/nu mice (10 per group) which received injections of 100μg of the trivalent synthetic antibody, $(LS2H)_2XCHA$, also referred to as Fab'3, on days 2 and 7 following injection of $10^7$ $2780^{AD}10$ ovarian carcinoma cells remained 70% tumor free for the duration of the experiment (210 days). Control mice, which received saline injections all developed tumors and died of disease by day 65. Figure 1 shows that mice which were injected with intact anti-p-glycoprotein monoclonal antibody, LS2H241 remained 90% tumor free and those mice which were injected with anti-p-glycoprotein antibody LS2L162 remained 100% tumor free for the duration of the experiment (210 days). Two irrelevant antibodies PSA399 and SI failed to protect mice from tumor development and mice in both of these groups developed tumors and died of disease similarly to the saline control group.

**EXAMPLE V**

INHIBITION OF MULTIDRUG-RESISTANT HUMAN OVARIAN CARCINOMA GROWTH IN ATHYMIC MICE BY LS2H241 Fab'2

The ability of the Fab'2 fragment of one of the anti-p-glycoprotein antibody LS2H241, referred to as LS2H241 Fab'$_2$ was also examined for its ability to block tumor growth in the human ovarian carcinoma xenograft model, 2780$^{AD}$10. Balb c/nu/nu mice were injected with 100μg of LS2H241 Fab'$_2$ on days 2 and 7 following s.c. inoculation of $10^7$ 2780$^{AD}$10 cells. Intact anti-p-glycoprotein monoclonal antibodies LS2H241 and LS2L162 as well as monoclonal antibodies PSA399, MOPC21 and BC4E549 were included as irrelevant controls. The BC4E549 antibody recognizes an antigen present on the 2780$^{AD}$10 cells. The mice which received LS2H241 Fab'2 failed to develop tumors for the duration of the experiment. The mice which received anti-p-glycoprotein monoclonal antibodies LS2L162 and LS2H241 remained 100% and 80% tumor free respectively. The results are shown in Figure 2.

**EXAMPLE VI**

LACK OF INHIBITION OF DRUG SENSITIVE HUMAN TUMOR GROWTH IN ATHYMIC MICE BY ANTI-P-GLYCOPROTEIN MONOCLONAL ANTIBODIES

In order to determine if the mechanism responsible for the tumor growth inhibition was related to the expression of p-glycoprotein on the surface of the tumor cells, the drug sensitive parental cell line A2780 was injected into nude mice and the effect of the anti-p-glycoprotein antibody, LS2L162, was studied. Figure 3 shows that injection of 500μg of either anti-p-glycoprotein monoclonal antibody LS2162 or the irrelevant antibody PSA399 had no effect on the growth of this drug sensitive tumor in nude mice.

**EXAMPLE VII**

INHIBITION OF GROWTH OF MULTIDRUG-RESISTANT HUMAN COLON CARCINOMA XENOGRAFTS IN NUDE MICE BY ANTI-P-GLYCOPROTEIN ANTIBODIES

In order to determine if the anti-tumor response of anti-p-glycoprotein antibodies was restricted to ovarian carcinomas or was applicable to other multidrug resistant tumors, the same in vivo experiments were carried out with the p-glycoprotein expressing colon carcinoma tumor, Colo320DM. Figure 4 shows that both anti-p-glycoprotein antibodies LS2H241 and LS2L162 are effective in inhibiting tumor growth in the Colo320DM colon carcinoma xenograft model.

The foregoing description of the invention is exemplary for purposes of illustration and explanation. It should be understood that various modifications can be made without departing from the spirit and scope of the invention. Accordingly, the following claims are intended to be interpreted to embrace all such modifications.

## Claims

1. A method for treating a patient having a disorder associated with tumor cells expressing a multidrug resistant p-glycoprotein, comprising administering to said patient a therapeutically effective amount of a specific binding agent having specific reactivity with said multidrug resistant p-glycoprotein, wherein said inhibition is non-complement or non-ADCC mediated.

2. The method of claim 1 wherein said specific binding agent is an antibody.

3. The method of claim 2, wherein said antibody is a monoclonal antibody.

4. The method of claim 3, wherein the monoclonal antibody is LS2H241.

5. The method of claim 1, wherein the specific binding agent is a fragment of an antibody.

6. The method of claim 5, wherein the specific binding agent is LS2H241F(ab)'$_2$.

7. The method of claim 1, wherein the specific binding agent is a trifunctional antibody.

8. The method of claim 7, wherein the trifunctional antibody is (LS2H241)$_2$XCHA.

9. Use of a specific binding agent having specific reactivity with a multidrug resistant p-glycoprotein expressed by tumor cells said inhibition being non-complement or non-ADCC mediated, for the manufacture of a medicament for treating a patient having a disorder associated with said tumor cells

10. Use according to claim 9 wherein the specific binding agent is as defined in any of claims 2 to 8.

FIG. I

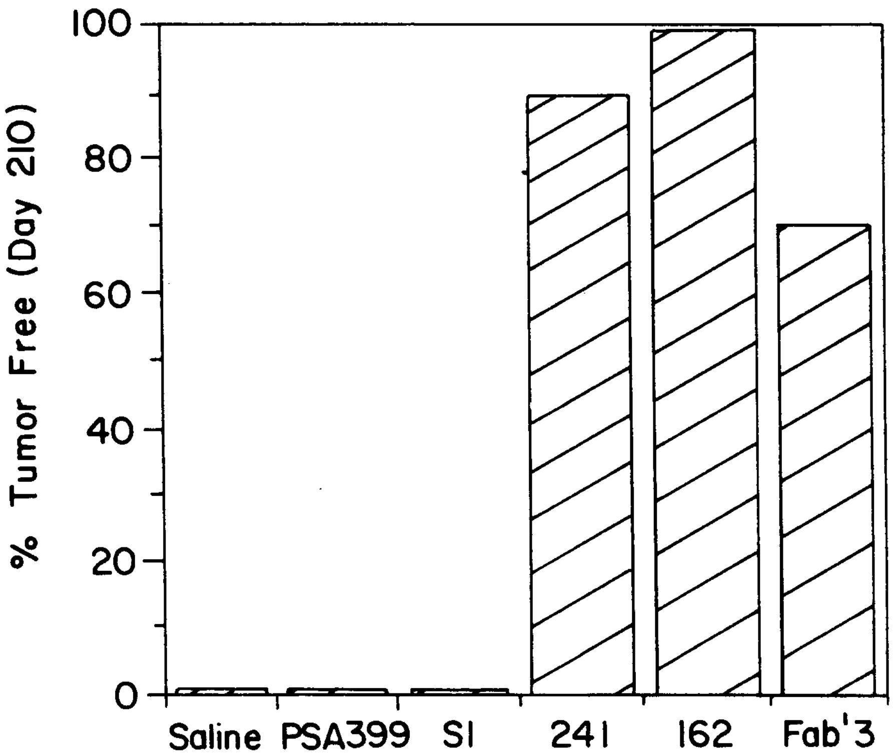
100
80
60
40
20
0
% Tumor Free (Day 210)
Saline
PSA399
SI
241
162
Fab'3

FIG. 2

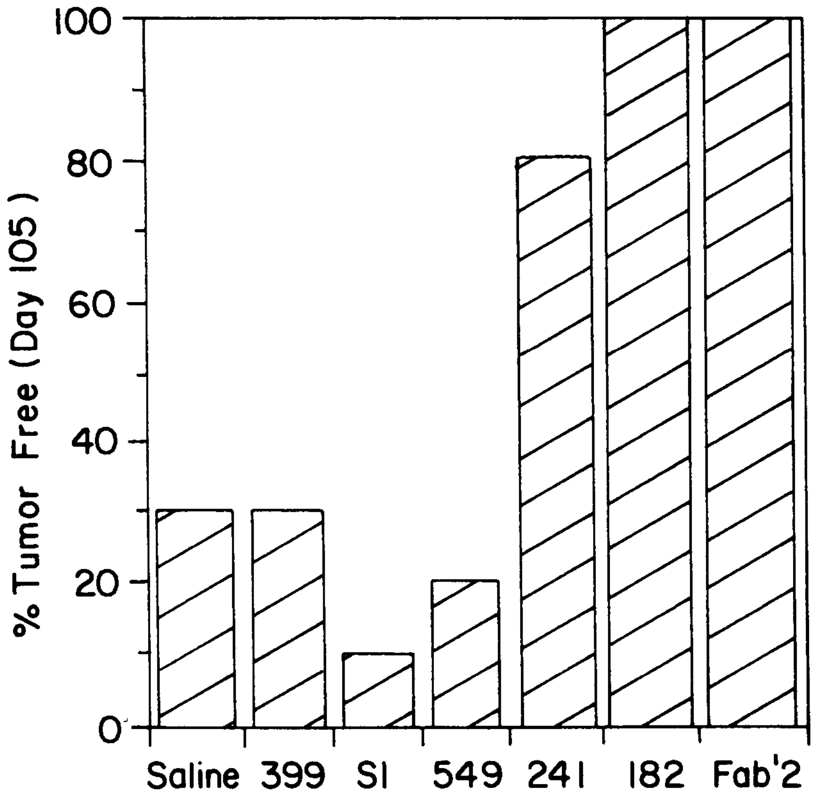
100
80
60
40
20
0
% Tumor Free (Day 105)
Saline
399
SI
549
241
182
Fab'2

FIG.3

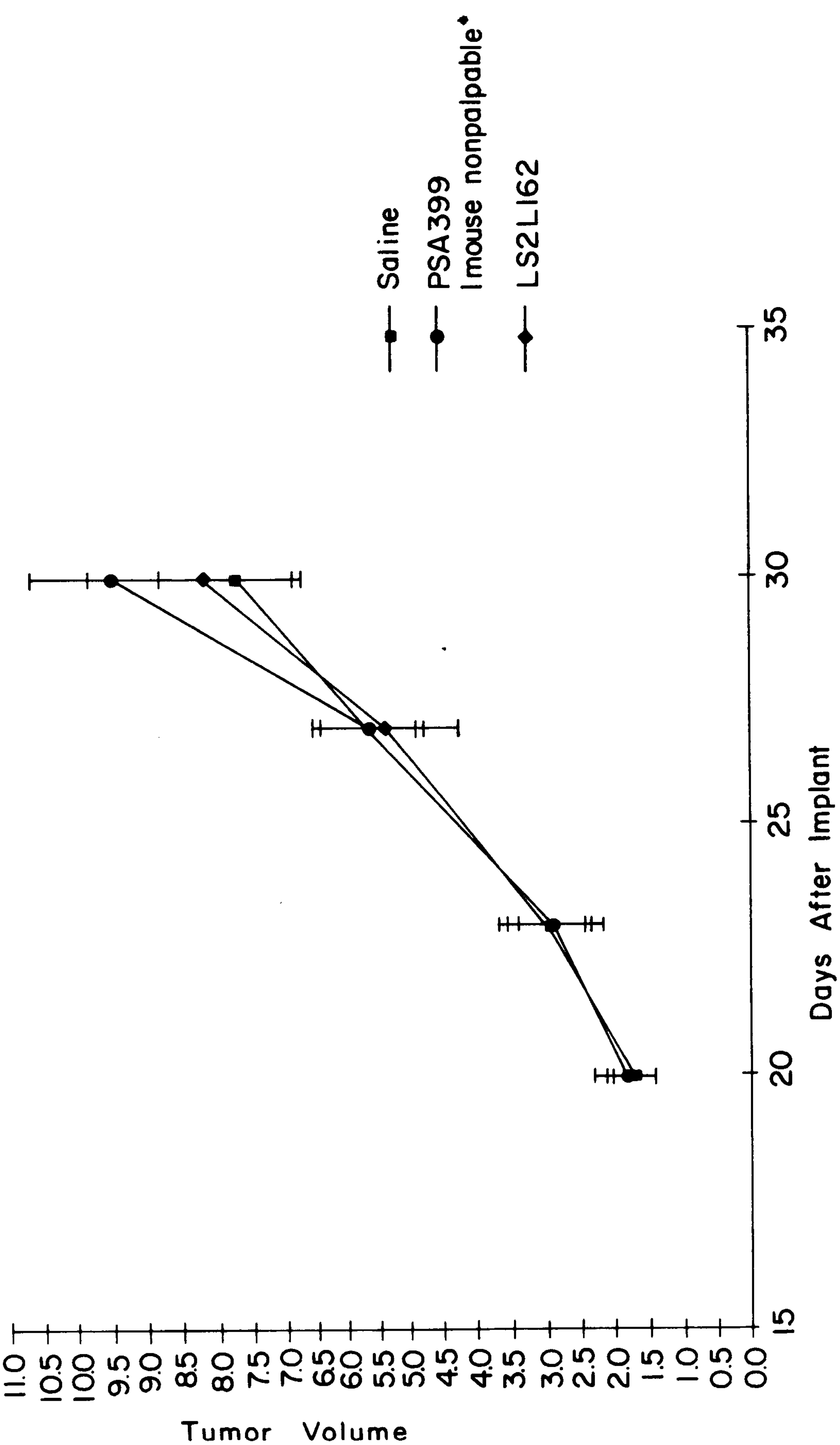

Saline
PSA399
1 mouse nonpalpable*
LS2L162
Tumor Volume
11.0
10.5
10.0
9.5
9.0
8.5
8.0
7.5
7.0
6.5
6.0
5.5
5.0
4.5
4.0
3.5
3.0
2.5
2.0
1.5
1.0
0.5
0.0
15
20
25
30
35
Days After Implant

FIG.4

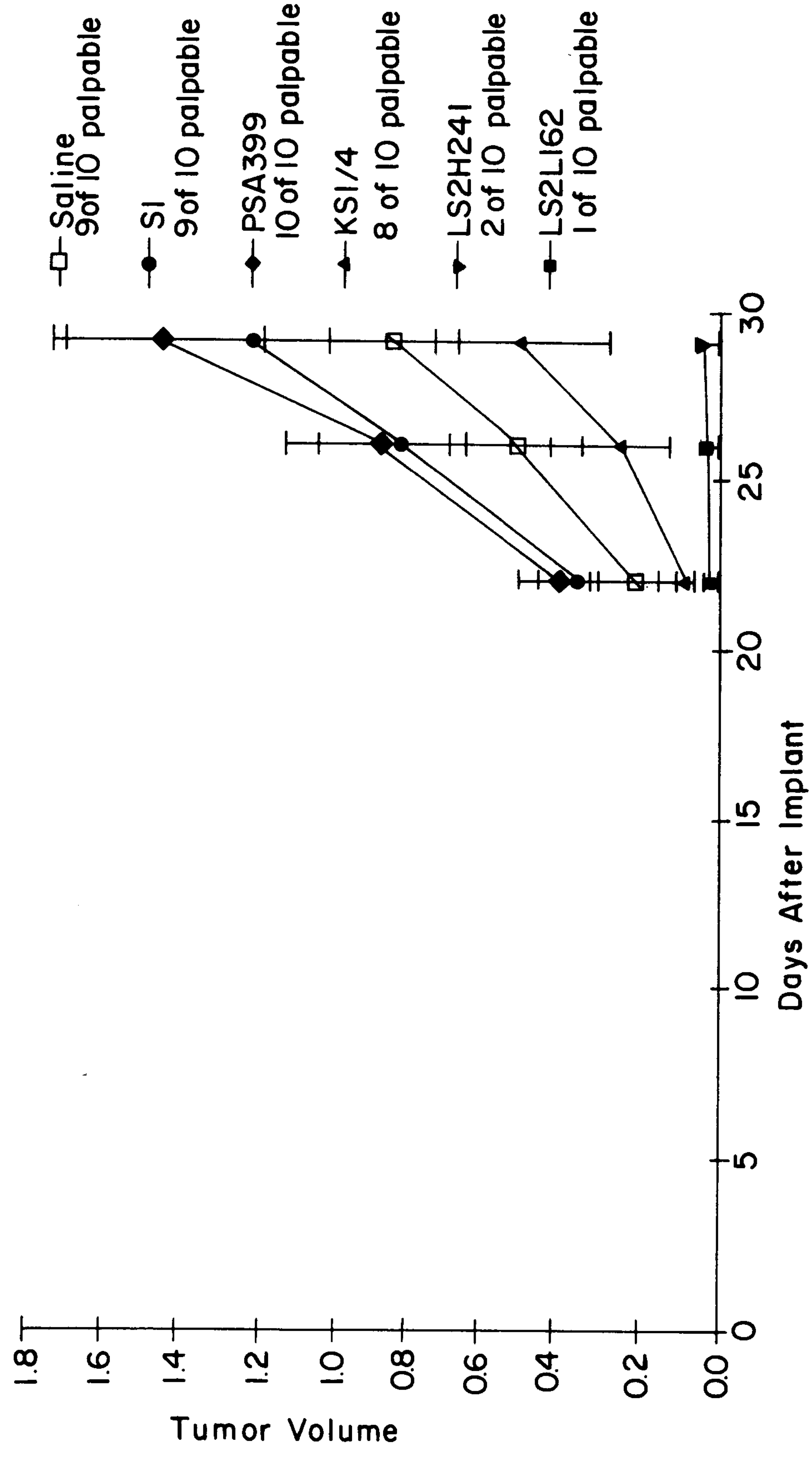
Saline 9of IO palpable
SI 9of IO palpable
PSA399 IO of IO palpable
KSI/4 8 of IO palpable
LS2H241 2 of IO palpable
LS2LI62 I of IO palpable
Tumor Volume
0.0
0.2
0.4
0.6
0.8
1.0
1.2
1.4
1.6
1.8
0
5
10
15
20
25
30
Days After Implant

FIG. 5

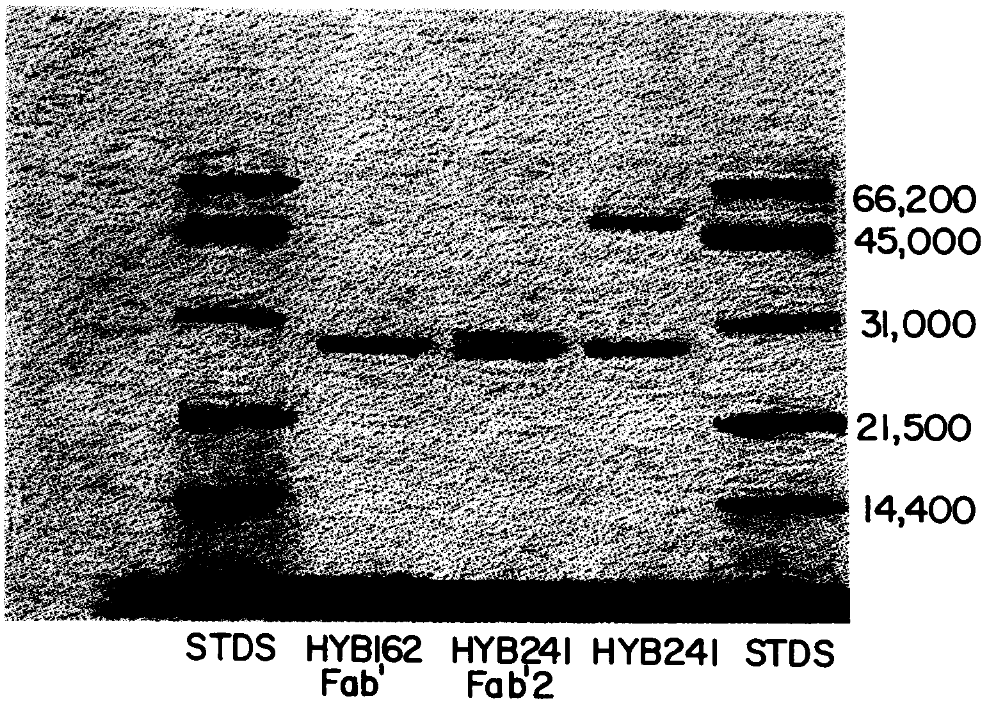
66,200
45,000
31,000
21,500
14,400
STDS
HYB162 Fab'
HYB241 Fab'2
HYB241
STDS

FIG. 6

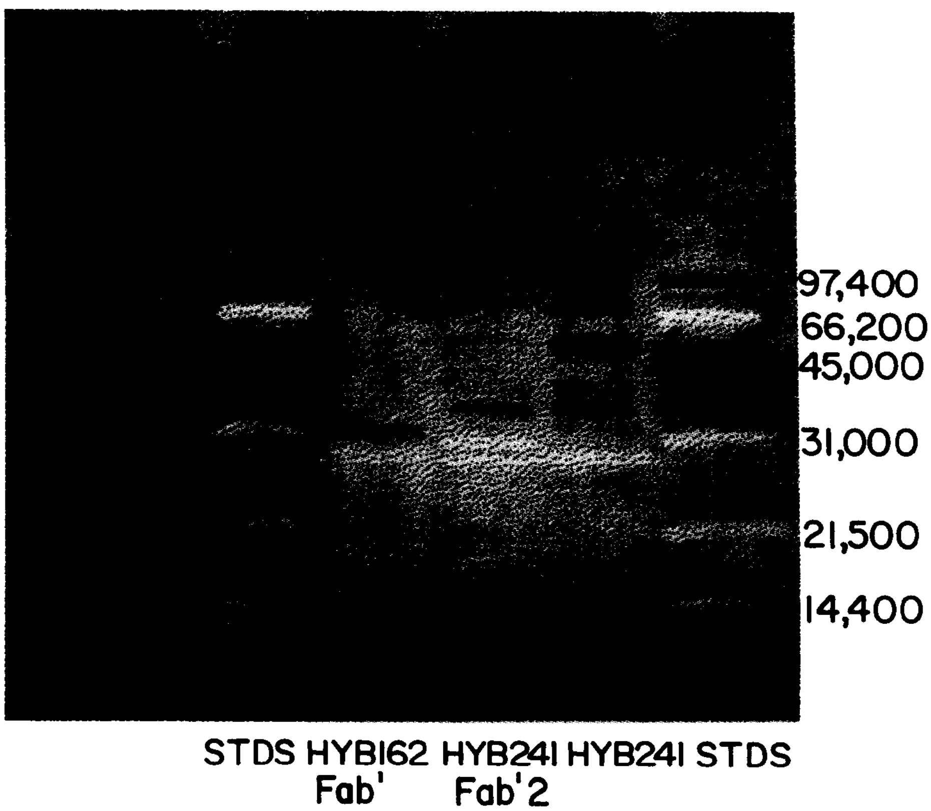
97,400
66,200
45,000
31,000
21,500
14,400
STDS
HYB162 Fab'
HYB241 Fab'2
HYB241
STDS